Europäisches Patentamt

European Patent Office

Office européen des brevets

(19)

(11) Publication number: **0 079 207**
A1

## (12) EUROPEAN PATENT APPLICATION

(21) Application number: 82305877.1

(22) Date of filing: 04.11.82

(51) Int. Cl.³: **C 07 C 1/12**
C 07 C 27/06, C 10 L 1/06

(30) Priority: 07.11.81 GB 8133639

(43) Date of publication of application:
18.05.83 Bulletin 83/20

(84) Designated Contracting States:
BE DE FR GB IT NL

(71) Applicant: The British Petroleum Company p.l.c.
Britannic House Moor Lane
London EC2Y 9BU(GB)

(72) Inventor: Ball, William, John
The British Petroleum Company p.l.c. Chertsey Road
Sunbury-on-Thames Middlesex, TW16 7LN(GB)

(72) Inventor: Stewart, David Gordon
The British Petroleum Company p.l.c. Chertsey Road
Sunbury-on-Thames Middlesex, TW16 7LN(GB)

(74) Representative: Harry, John et al,
BP INTERNATIONAL LIMITED Patents and Licensing
Division Chertsey Road
Sunbury-on-Thames Middlesex TW16 7LN(GB)

(54) Process for the production of hydrocarbons and oxygenated derivatives thereof by the catalytic hydrogenation of carbon dioxide.

(57) Hydrocarbons and their oxygenated derivatives are produced by reacting a mixture essentially comprising carbon dioxide and hydrogen at elevated temperature and pressure in the presence of a catalyst comprising supported rhodium, preferably in combination with one or more of the metals chromium, molybdenum, silver and zirconium. The preferred support is silica.

1

## PROCESS FOR THE PRODUCTION OF HYDROCARBONS AND OXYGENATED DERIVATIVES THEREOF BY THE CATALYTIC HYDROGENATION OF CARBON DIOXIDE

The present invention relates to a process for the production of hydrocarbons and oxygenated derivatives thereof from a mixture of carbon dioxide and hydrogen and in particular to the production of a mixture of alcohols principally comprising methanol and ethanol from a mixture of carbon dioxide and hydrogen.

Alcohols are gaining increasing importance as gasoline supplements and automotive fuels. In some countries there are plans for satisfying the increased demand by fermentation of natural products, eg molasses and beet, but worldwide there is a growing recognition of the need for an inexpensive mixed alcohol process. In this context the catalytic conversion of synthesis gas (mixtures of carbon monoxide and hydrogen), which can be obtained from a variety of sources such as oil, coal and natural gas, has been studied extensively, as is evidenced by a rapidly growing patents literature. Recent patent publications describe the production of oxygenated hydrocarbons from synthesis gas using as catalyst rhodium (US Patent No 4246186), rhodium/iron (US Patent No 4235801), rhodium/manganese (USP 4014913), rhodium/molybdenum or rhodium/tungsten (USP 4096164), rhodium/ruthenium (USP 4101450), rhodium/uranium/thorium (USP 4162262), rhodium/chromium (European patent publication No 18763), rhodium/zirconium (European patent publication No. 30110) and rhodium/rhenium (UK patent publication No. 2078745). Surprisingly, in view of the abundance of carbon dioxide and its formation as a byproduct of synthesis gas conversion, relatively little attention appears to have been paid to the catalytic conversion of mixtures of carbon dioxide and hydrogen to hydrocarbons and oxygenated derivatives thereof. Certain of the aforesaid patents, eg USP 4235801 and USP 4235798, relating to synthesis gas conversion mention that carbon dioxide, normally present in an amount up to about 10 mole per cent, has essentially no effect upon the reaction.

We have now found that mixtures of carbon dioxide and hydrogen can be converted to hydrocarbons and oxygenated derivatives thereof, such as a mixture of alcohols principally comprising methanol and ethanol, by reaction over a supported rhodium catalyst.

Accordingly the present invention provides a process for the production of hydrocarbons and oxygenated derivatives thereof which process comprises reacting a mixture essentially comprising carbon dioxide and hydrogen at elevated temperature and pressure in the presence of a catalyst comprising supported rhodium.

Mixtures of carbon dioxide and hydrogen are abundantly available as a product of the reaction of steam with carbon monoxide which itself is obtainable from the reaction of steam with carbonaceous matter, the catalytic steam reforming of methane and partial oxidation of carbonaceous matter. Carbon dioxide is also produced by the decomposition of limestone and by alcoholic fermentation. A suitable source of carbon dioxide is the carbon dioxide separated from the reaction product resulting from the hydrogenation of carbon monoxide in the presence of rhodium-containing catalysts. Although it is preferred to employ substantially pure carbon dioxide and hydrogen, the presence of impurities such as carbon monoxide and nitrogen can be tolerated. For example, carbon monoxide may be present in an amount up to 10 mole per cent. On the other hand impurities which have a deleterious effect on the reaction should be avoided. The ratio of hydrogen to carbon dioxide may vary over a moderately wide range. Normally the molar ratio of hydrogen to carbon dioxide may be in the range of from 10:1 to 1:10, preferably from 5:1 to 1:5.

A wide variety of support materials may be employed. Suitable support materials include silica, alumina, silica/alumina, magnesia, thoria, titania, chromia, zirconia and active carbon, of which silica is preferred. Zeolite molecular sieves and in particular the crystalline zeolites may be employed. Suitably the support has a relatively high surface area. The support may have a surface area up to 350 square metres per gram (BET low temperature nitrogen adsorption isotherm method), preferably in the range 1 to 300 square metres per gram. Whilst the actual form of the rhodium under the reaction conditions is not known with any degree of certainty it is likely that it is in either the oxide form or in the metallic form under the reducing conditions prevailing. Thus the rhodium may be added in the form of the elemental metal or in the form of rhodium compounds. The rhodium may be deposited on the support by any of the techniques commonly used for catalyst preparation. Although it is possible to add particles of the rhodium metal to the support it is preferred to use the techniques of impregnation from an organic or inorganic solution, precipitation, coprecipitation or cation exchange.

Conveniently the catalyst may be prepared by impregnating the support with a solution of inorganic or organic rhodium compounds. Suitable compounds are rhodium salts e.g. the halides, particularly the chlorides and nitrates. Following impregnation the catalyst is preferably dried and heated in a reducing atmosphere eg hydrogen. The amount of rhodium on the support may suitably be in the range of from 0.01 to 25 weight percent, preferably from 0.1 to 10 weight percent, based on the combined weight of the metals and the support. The catalyst may be further improved by incorporating on the support one or more other metals selected from Groups I to VIII of the Periodic Table. Suitable metals include iron, manganese, rhenium, zirconium, tungsten, molybdenum, ruthenium, chromium, silver, thorium, uranium and potassium. One or more of the metals chromium, molybdenum, silver and zirconium are particularly preferred. Each additional metal component may be present in an amount in the range from 0.1 to 10 weight percent based on the combined weight of the metals and the support.

In another embodiment of the present invention the support can be activated by the addition of one or more metal or non-metal activator components followed by calcination prior to incorporation of the rhodium, and, optionally, other metals. Whilst a wide variety of such

0079207

metals and non-metals may be added, the alkali metals, thorium, manganese, rhodium, iron, chromium, molybdenum, zirconium, rhenium, silver, boron and phosphorus are specific examples of such materials. Any of the known techniques for catalyst preparation hereinbefore referred to may be used for addition of the activating material. In the case of a metal activator the support is preferably impregnated with a solution of a compound of the metal, suitably the nitrate or chloride, and is thereafter dried, suitably by evaporation and calcined. The activated support is then in a suitable condition for addition of the rhodium and optionally, other metals. The amount of activator component added may suitably be in the range 0.01 to 50 weight percent, preferably from 1 to 25 weight percent based on the combined weight of the activator component and the support.

With regard to the reaction conditions the temperature is preferably in the range from 200 to 400°C and even more preferably from 220 to 350°C; the use of higher temperatures within the aforesaid ranges tends to increase the co-production of methane. Because of the highly exothermic nature of the reaction the temperature requires careful control in order to prevent a runaway methanation, in which methane formation increases with increasing temperature and the resulting exotherm increases the temperature still further. In fixed bed operations, temperature control may be achieved by mixing the catalyst with an inert diluent, thereby ensuring that the exothermic heat is more evenly distributed. In this way the useful life of the catalyst may be protected and prolonged. The reaction pressure is preferably in the range from 20 to 300 bars. The use of higher pressures within the aforesaid ranges increases the production rate and selectivity to oxygenated hydrocarbons.

An important reaction parameter is the gas hourly space velocity (volume of gas, at NTP, per volume of catalyst per hour). Suitably the gas hourly space velocity is greater than $10^3$ per hour. Excessively high space velocities result in an uneconomically low conversion while excessively low space velocities result in a loss of selectivity to desirable products.

Although the reaction may be carried out batchwise it is preferably carried out in a continuous manner.

The effluent from the reaction may be freed from the desired oxygenated products by various means, such as scrubbing and/or distillation. The residual gas which consists mainly of unreacted carbon dioxide and hydrogen may be mixed with fresh carbon dioxide and/or hydrogen to give the required reactor feed and this composite gas then recycled to the reactor inlet.

The oxygenated hydrocarbon compounds principally comprise a mixture of $C_1$ to $C_3$ alcohols, ie methanol, ethanol and propanol, in which $C_1$ and $C_2$ alcohols predominate. The relative proportions of the $C_1$ to $C_3$ alcohols may be altered if desired by incorporating one or more metallic components into the catalyst and/or by altering the relative proportions of the metals in the catalyst and/or by varying the nature of the support.

As hereinbefore mentioned oxygenated hydrocarbons such as mixtures of alcohols are useful as gasoline supplements.

In another aspect therefore the present invention provides an internal combustion engine fuel composition comprising a major proportion of gasoline and a minor proportion of the oxygenated hydrocarbons produced by the process as hereinbefore described.

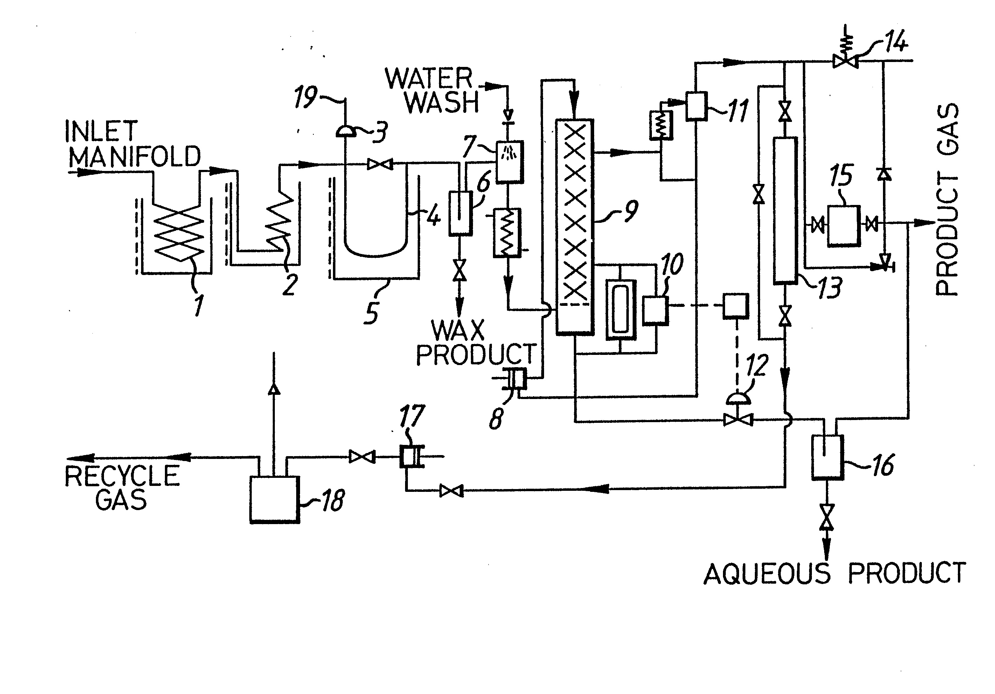

The process of the invention will now be illustrated by the following Examples and by reference to the accompanying Figure which is a simplified flow diagram of the apparatus employed.

With reference to the Figure, 1 is a preheater (150°C), 2 is a preheater (200°C), 3 is a bursting disc, 4 is a reactor, 5 is a salt pot, 6 is a knock-out pot, 7 is a water quench, 8 is a water recycle pump, 9 is a water wash tower, 10 is a DP level controller, 11 is a knock-out pot, 12 is a Foxboro valve, 13 is a molecular sieve drier, 14 is a Gyp relief valve, 15 is a back pressure regulator, 16 is an aqueous product receiver, 17 is a gas recycle pump, 18 is a ballast vessel and 19 is a vent.

Also in the Examples the terms $CO_2$ conversion and selectivity will be used. For the avoidance of doubt these are defined as follows:

$$CO_2 \text{ conversion} = \frac{\underline{\text{Moles of carbon dioxide consumed}} \times 100}{\text{Moles of carbon dioxide fed}}$$

$$\text{Selectivity} = \frac{\text{Moles of carbon dioxide converted to}}{\text{Moles of carbon dioxide consumed}} \times 100$$

## Catalyst (rhodium/silver/zirconium/molybdenum)

Silver nitrate (2.7 g) was dissolved in deionised water (50 ml) and the solution added to Davison silica, grade 59 (30 g, 8-16 mesh granules). The mixture was evaporated to dryness on a steam bath and the dry product reduced in hydrogen at 450°C for 6 hours.

Zirconium tetrachloride (3.9 g) and rhodium trichloride trihydrate (3.9 g) were dissolved in deionised water (50 ml) and the resulting solution added to the silver-silica support. The mixture was evaporated to dryness on a steam-bath and the dry product reduced in hydrogen at 450°C for 6 hours.

Ammonium heptamolybdate tetrahydrate (1.26 g) was dissolved in deionised water (50 ml) and the resulting solution added to the above treated support. The mixture was evaporated to dryness on a steam bath and the dry product heated at 450°C in hydrogen for 6 hours.

## Catalyst Testing

### Example 1

With reference to the accompanying Figure a mixture of carbon dioxide and hydrogen in a molar ratio of 1:2 was passed via the inlet manifold through the two preheater coils (1) and (2) maintained at 150°C and 200°C respectively in silicone oil baths. The heated gases were then fed via a heat-traced line to the copper lined reactor (4) which was maintained at 50 bars pressure and contained a fixed bed of the rhodium/silver/zirconium/molybdenum/silica catalyst in the form of 8-16 mesh (BSS) granules. The reactor was maintained at the desired reaction temperature by immersion in a molten salt bath (5). The product gases were passed via a heat-traced line through a knock-out pot for wax products (6) to a small quench vessel (7) into the top of which water was sprayed. The gases were then passed through a water cooler to the bottom of the water wash tower (9) which was packed with 3/8 inch Raschig rings. In the tower (9) the product gases were washed counter-current with water. The resulting liquid product was fed into the receiver (16) and any dissolved gases were recombined with the product gas stream from the back pressure regulator (15). The separated gas stream from the top of the water wash tower (9) was passed through a water cooler to the knock-out pot (11) and then to the inlet side of the dome-loaded back pressure regulator (15). Recycle gas was recovered from the inlet side of the back pressure regulator (15), passed through a molecular sieve drier (13) and compressed up to 67 bars in the gas ballast vessel (18) using the gas recycle pump (17). The recycle gas was fed back to the inlet manifold. Provision was made to feed spot samples of the inlet gases and the total gas stream to a gas chromatographic analytical unit.

The product gas stream leaving the back pressure regulator (15) was measured and samples were withdrawn and analysed by gas chromatography.

When the reactor had reached equilibrium a balanced run was carried out over a one hour period.

The reaction conditions and the results obtained are given in the accompanying Table.

## TABLE

Feed : $CO_2$ : $H_2$ = 1:2 (molar)
Pressure : 50 bar

| Run | Temp. °C | Recycle ratio | GHSV $hr^{-1}$ | $CO_2$ Conv. % | Molar Selectivity % | | | | | | Productivity (gmol/hr/ 1 cat) |
|---|---|---|---|---|---|---|---|---|---|---|---|
| | | | | | CO | $CH_4$ | $C_2$'s | $C_3$'s | MeOH | Et.OH | |
| 5196/11 | 260 | NONE | 1286 | 28.1 | 42.3 | 34.6 | 1.7 | – | 18.8 | 2.6 | 1.1 |
| 5196/12 | 260 | 4.7:1 | 7200 | 33.6 | 29.3 | 38.0 | 1.8 | – | 27.7 | 3.1 | 1.9 |
| 5196/10 | 260 | 15:1 | 18947 | 42.0 | 16.5 | 37.4 | 1.9 | 0.3 | 40.3 | 3.6 | 3.3 |

0079207

Claims:

1. A process for the production of hydrocarbons and oxygenated derivatives thereof which process comprises reacting a mixture essentially comprising carbon dioxide and hydrogen at elevated temperature and pressure in the presence of a catalyst comprising supported rhodium.

2. A process according to claim 1 wherein the support is silica.

3. A process according to either claim 1 or claim 2 wherein there is also incorporated on the support one or more of the metals iron, manganese, rhenium, zirconium, tungsten, molybdenum, ruthenium, chromium, silver, thorium, uranium and potassium.

4. A process according to claim 3 wherein there is also incorporated on the support one or more of the metals chromium, molybdenum, silver and zirconium.

5. A process according to any one of the previous claims wherein the elevated temperature is in the range 200 to 400°C and the elevated pressure is in the range 20 to 300 bars.

6. A process according to claim 5 wherein the temperature is in the range 220 to 350°C.

7. A process according to any one of the preceding claims wherein the gas hourly space velocity is greater than $10^3$ per hour.

8. An internal combustion engine fuel composition comprising a major proportion of gasoline and a minor proportion of the oxygenated hydrocarbons produced by the process as claimed in any one of claims 1 to 7.

PRODUCT GAS

AQUEOUS PRODUCT

WATER WASH

WAX PRODUCT

INLET MANIFOLD

RECYCLE GAS

## European Patent Office

**EUROPEAN SEARCH REPORT**

**0079207**
Application number

EP  82 30 5877

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | C 07 C    1/12 |
| X | CHEMICAL ABSTRACTS, vol. 91, no. 2, 9th July 1979, page 320, no. 10046n, Columbus, Ohio, USA & JP - A - 79 41291 (SAGAMI CHEMICAL  RESEARCH CENTER) 02-04-1979 * Abstract * | 1 | C 07 C   27/06 C 10 L    1/06 |
| | --- | | |
| X | WO-A-7 900 290  (JOHNSON, MATTHEY & CO.) * Claims * | 1-3,5, 6 | |
| | --- | | |
| X | US-A-3 787 468  (D.K. FLEMING et al.) * Examples 6-9 * | 1,3,5, 6 | |
| | --- | | |
| X | CHEMICAL ABSTRACTS, vol. 94, no. 4, 26th January 1981, page 361, no. 21064e, Columbus, Ohio, USA F.   SOLYMOSI   et   al.: "Hydrogenation  of carbon dioxide to methane over alumina-supported noble  metals" & MAGY. KEM. FOLY. 1980,  86(10),  476-478 * Abstract * | 1 | **TECHNICAL FIELDS SEARCHED (Int. Cl. ³)** C 07 C    1/00 C 07 C   29/00 C 07 C   27/00 |
| | --- | | |
| A | FR-A-2 295 118  (H. BRUSSET) * Claims 5-8 * | | |
| | ----- | | |

The present search report has been drawn up for all claims

| Place of search THE HAGUE | Date of completion of the search 04-02-1983 | Examiner VAN GEYT J.J.A. |
|---|---|---|